# EUROPEAN PATENT APPLICATION

(11) **EP 1 223 149 A1**
(43) Date of publication of application: **17.07.2002**
(21) Application number: 01400052.5
(22) Date of filing: 10.01.2001
(51) Int. Cl.: C03C 17/30, C07H 21/00

(54) **Silsesquioxane-coated substrates for immobilizing biomolecules**

(71) Applicant: Corning Incorporated, Corning, New York 14831 (US)
(72) Inventor: Laguitton, Bruno, Corning, New York 14831 (US)
(74) Representative: Marchant, James Ian

(57) **Abstract**

A method of coating a substrate for immobilizing a biomolecule and substrates produced by the method containing an immobilized biomolecule. The method includes coating a substrate with a silsesquioxane and immobilizing a biomolecule on the substrate.

## Description

### FIELD OF THE INVENTION

This invention relates to substrates for use in immobilizing biomolecules and methods of making such substrates. More particularly, the present invention relates to silsesquioxane-coated substrates and methods of fabricating such coated substrates.

### BACKGROUND OF THE INVENTION

Analysis of the structure, organization and sequence of nucleic acid molecules is important in the prediction, diagnosis and treatment of human disease and in the study of gene discovery, expression and development. One laboratory tool used in the analysis of nucleic acid molecules is the high density array (HDA). The HDA provides the framework for immobilization of nucleic acid molecules for analysis on a rapid. large-scale basis. HDAs generally include a substrate having a large number of positionally distinct DNA probes attached to a surface of the substrate for subsequent hybridization to a DNA target. The key to efficiently immobilizing nucleic acid molecules is the surface chemistry and the surface morphology of the HDA substrate.

The surfaces of both organic and inorganic substrates can be modified by the deposition of a polymeric monolayer coating or film to construct biomolecular assemblies. In addition. surface modification can also be used to promote adhesion and lubrication. modiy the electrical and optical properties of the substrate surface. and create electroactive films suitable for various optical and electronic sensors and devices.

Monolayer films are limited in that they are not capable of being modified to achieve a desired surface characteristic such as uniform electrical charge with a precisely controlled thickness and molecular organization. To obtain this desired surface characteristic, a multi-layered film having a well-defined molecular organization in a smooth geometrical arrangement must be produced on substrate, using either the Langmuir-Blodgett method or other known methods. However. these methods have certain limitations. For example, the Langmuir-Blodgett method produces films that tend to be mechanically unstable.

Compounds with amine functionality have found extensive application in the preparation of surfaces for nucleic acid hybridization. Due to their ability to bond to a substrate with a hydroxide and their ability to bond to nucleic acids with an amine. silane compounds are useful as surface coatings that will effectively immobilize nucleic acids. One example of a silane used for biological assay preparation is gamma amino propyl silane (GAPS). which may be deposited by a variety of methods, including CVD. spin coating, spray coating and dip coating.

One limitation of GAPS coatings is that substrates coated with GAPS using certain methods are relatively expensive and difficult to prepare. Another limitation of certain GAPS-coated slides is that they do not tend to exhibit long-term durability. possibly due incomplete reaction of the GAPS molecules. Although improvements have been made in GAPS coating procedures, it would be desirable to provide alternate coating techniques.

A very important consideration in the preparation of substrates for immobilization of biomolecules is uniformity of the coating on the substrate. It is insufficient to provide a substrate with partial functionality. It is important to provide uniform functionality over an extended area of the substrate. This is especially true in the case of high density arrays for performing biomolecule hybridization assays. Such assays rely on having uniform levels of biomolecule immobilization at known locations on the substrate. It is desirable to have substantially identically sized spots containing a known quantity of pre-determined set of capture biomolecules located on the substrate in a regular geometric array with low background. Ambiguous and/or erroneous readouts result from variations in the immobilization and localization of the capture biomolecules.

It would be useful to provide a relatively simple coating and method for applying a coating to a substrate for immobilization and hybridization of biomolecules such as nucleic acids and oligonucleotides. The coating and the method of applying the coating should have the ability to be applied in a reproducible manner. It would also be advantageous to provide a coating that has uniform surface characteristics.

### SUMMARY OF INVENTION

Accordingly, the present invention generally provides a method of immobilizing biomolecules on a surface of a substrate. The method involves coating the substrate with a silsesquioxane solution and immobilizing the biomolecules on the silsesquioxane-coated surface. According to one aspect of the invention, the silsesquioxane is a silsesquioxane oligomer. In another aspect of the invention, the silsesqioxane oligomer is in an aqueous solution.

According to a preferred aspect, the silsesquioxane oligomer has an average molecular weight of from about 150 to about 1000. More preferably, the silsesquioxane oligomer has an average molecular weight of from about 200 to about 700. In a highly preferred aspect, the silsesquioxane oligomer has an average molecular weight ranging of from about 250 to about 650.

Preferably, the oligomer used for the coating of the present invention, is an aminoalkylsilsesquioxane. including a C₂ or higher alkyl group. According to one preferred aspect, the oligomer is an aminopropylsilsesquioxane oligomer. According to another aspect, the oligomer is an aminopropylsilsesquioxane-methylsilsesquioxane copolymer oligomer.

Another aspect of the invention pertains to a substrate including a coating of a polysilsesquioxane and having biomolecules immobilized thereon. Preferably, the coating is an aminoalkylsilsesquioxane. including a C₂ or higher alkyl group. For example, the aminoalkylsilsesquioxane may be aminopropylsilsesquioxane oligomer. aminopropylsilsesquioxane-methylsilsesquioxane copolymer oligomer. or a mixture thereof.

According to one aspect of the invention, the immobilized biomolecules are nucleic acid molecules or oligonucleotides. In a preferred aspect of the invention, the substrate is a high density array or microplate.

The present invention provides a simplified and reproducible method of providing substrates for immobilizing biomolecules. Experimentation has indicated that the substrates produced according to the invention have good stability. Additional features and advantages of the invention will be set forth in the following description. It is to be understood that both the foregoing general description and the following detailed description are exemplary and are intended to provide further explanation of the invention as claimed.

### BRIEF DESCRIPTION OF THE DRAWINGS

FIG. 1 is a graph comparing the average relative fluorescence unit (Rfu) signals of hybridization experiments using slides coated with silsesquioxanes and GAPS.

FIG. 2 is a chart depicting comparative fluorescence imaging of three different sets of slides coated with silsesquioxane oligomers treated at two different temperatures compared with slides coated with GAPS.

FIG. 3 is a statistical graph analyzing the uniformity of the spot size produced on slides coated with silsesquioxanes according to the present invention.

### DETAILED DESCRIPTION

Reference will now be made in detail to the present preferred embodiment of the invention. The invention provides a method of coating substrates for immobilization of a biomolecule and substrates produced by the method have a biomolecule immobilized thereon.

According to the present invention, biomolecules are immobilized on a surface of a substrate coated with a silsesquioxane solution. Silsesquioxanes have the general formula RSiO_{1.5}, wherein R is a functional groups bonded to the silsesquioxane structure, such as for example, alkyl. amino. aminoalkyl. hydroxy. aminoalkylaminoalkyl and other functional groups appropriate for immobilizing biomolecules on a substrate coated with the silsesquioxane. When the substrate used for immobilizing biomolecules is a glass substrate, it is preferred that hydroxy functional groups are present. Amine moieties are also preferably present to provide interaction with DNA and other biomolecules. Other chemical groups can also be present so long as the resulting oligomer is soluble in a solvent, preferably a so that the oligomer is water soluble. According to a preferred aspect of the invention, the silsesquioxane coating is a silsesquioxane oligomer or polymer or mixtures thereof.

According to one preferred aspect of the invention, a silsesquioxane used to coat a substrate is described by the formula: 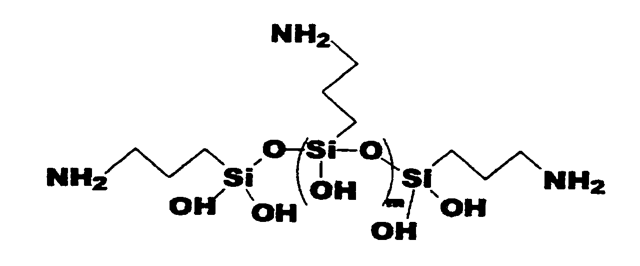 wherein, m is an integer greater than or equal to 1 such that the average molecular weight of a resulting silsesquioxane oligomer ranges from about 150 to about 1000.

Another preferred aspect of the invention, involves coating a substrate for immobilization of a biomolecule with a silsesquioxane described by the formula: 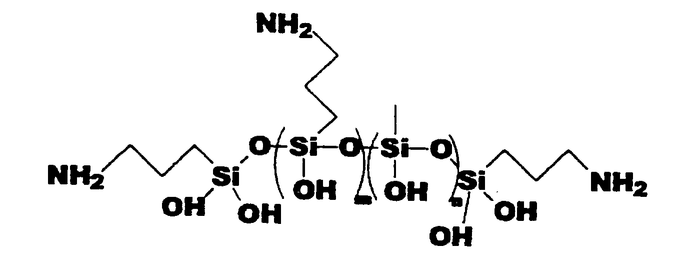 wherein m and n are integers greater than or equal to 1 such that the average molecular weight of the resulting silsesquioxane oligomer ranges from about 150 to about 1000.

According to still another preferred aspect of the invention, a polysilsesqioxane is used to coat a substrate for immobilization of a biomolecule. the polysilsesquioxane being described by the formula: 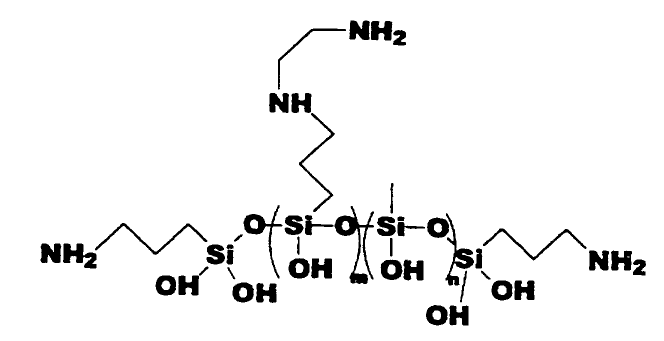 wherein m and n are integers greater than or equal to 1 such that the average molecular weight of the resulting silsesquioxane oligomer ranges from about 150 to about 1000.

As noted above, preferably the silsesquioxanes are silsesquioxane oligomers. Preferably, functional groups are attached to the Si atom of the silsesquioxane backbone and the number of monomer units that form the oligomer are selected such a substrate coated by a solution of the silsesquioxane oligomer is adapted for use in immobilizing biomolecules such as. for example, nucleic acids and oligonucleotides.

Particularly preferred silsesquioxanes are aminoalkylsilsesquioxanes, wherein the alkyl group is a C₂ or higher alkyl group. For example, one preferred oligomer is an aminopropylsilsesquioxane oligomer. Another preferred oligomer is an aminopropylsilsesquioxane-methylsilsesquioxane copolymer oligomer.

The silsesquioxane oligomers used to coat substrates for immobilization of biomolecules in accordance with the present invention should have an average molecular weight such that the silsesquioxane polymer is adapted to prepare a solution for forming a coating on a substrate for the immobilization of biomolecules. Preferably. the silsesquioxane oligomer has an average molecular weight of from about 150 to about 1000. and more preferably of from about 200 to about 700. In a highly preferred aspect of the invention. the silsesquioxane oligomer has an average molecular weight of from about 250 to about 650. Silsesquioxane oligomers appropriate for coating substrates for immobilizing biomolecules are available from Gelest. Inc.. in Tullytown, Pennsylvania.

Preferably, the silsesquioxane coating is applied in an aqueous solution by spraying, dipping, coating, brushing and other methods that can form a uniform and reproducible coating on a substrate used for immobilizing biomolecules, such as high density arrays and microplates. which can be made from a variety of materials. Such substrate materials include, but are not limited to glass, quartz or silica.

Suitable substrates for this invention are those having a surface that is accessible to solvents. The substrate itself may take any shape including, but not limited to. rectangular, square, circular, cylindrical, conical, planar and spherical. The interior surface of a bottle or tubing could be used as a substrate. The preferred substrate has a planar shape, and may be formed into a variety of HDAs. microplates and laboratory dishes.

For optical or electrical areas of application, the substrate can be transparent, impermeable or reflecting, as well as electrically conducting, semiconducting or insulating. For biological applications, the substrate material may be either porous or nonporous and may be selected from either organic or inorganic materials.

Inorganic substrate materials can include metals, semiconductor materials, glass and ceramic materials. Examples of metals that can be used as substrate materials are gold. platinum, nickel, palladium, aluminum, chromium, steel and gallium arsenide. Semiconductor materials used for the substrate material can include silicon and germanium. Glass and ceramic materials used for the substrate material can include quartz, glass, porcelain, alkaline earth aluminoborosilicate glass and other mixed oxides. Further examples of inorganic substrate materials include graphite, zinc selenide. mica, silica, lithium niobate. and inorganic single crystal materials.

Organic substrate materials are typically made from polymer materials, due to their dimensional stability and resistance to solvents. Examples of organic substrate materials are polyesters, such as polyethylene terephthalate. and polybutylene terephthalate, polyvinylchloride, polyvinylidene fluoride. polytetrafluoroethylene, polycarbonate, polyamide, poly(meth)acrylate, polystyrene, polyethylene or ethylene/vinyl acetate copolymer.

Once a suitable substrate is obtained, a silsesquioxane is coated on the surface of the substrate by using various techniques. A simple method of forming a coating is by dipping the substrate in an aqueous solution containing a water-soluble polysilsesquioxane.

According to one embodiment of the invention. DNA or oligonucleotides are attached to the silsesquioxane coated substrate. Other biological or synthetic molecules can be attached to the coated substrate. For example, other synthetic molecules include. but are not limited to. ribonucleic acids (RNA). deoxyribonucleic acids (DNA). synthetic oligonucleotides. antibodies. proteins. peptides, lectins, modified polysaccharides. synthetic composite macromolecules. functionalized nanostructures. synthetic polymers, modified/blocked nucleotides/nucleosides. modified/blocked amino acids, fluorophores, chromophores. ligands. chelates. and haptens.

To facilitate immobilization of biomolecules, it is desirable for the silsesquioxane used in coating the substrate to include one of various functional groups. These functional groups may include, but are not limited to. primary amines, propyl hydrocarbon chain segments, silanol groups and siloxane bonds. Generally. immobilization of molecules at a substrate surface occurs in two steps: attraction of the molecules to the surface and binding of the molecules to the surface. Some or all of the functional groups exposed on the surface of the silsesquioxane coating may contribute the attraction and binding of biomolecules or biomaterials. resulting in their immobilization on the substrate. For example, a protonated primary amine is positively charged and may charge-attract and bind biomolecules. Propyl hydrocarbon chains are hydrophobic. and their hydrophobic interaction with hydrophobic segments of biomolecules may assist in binding them to the surface. Other interactions between biomolecules and silsesquioxane-coated substrates are. of course, possible and the above discussion is not intended to be exhaustive or limiting of the mechanisms, which may play a role in the immobilization of biomolecules on silsesquioxane-coated substrates in accordance with this invention.

According to method aspects of the present invention, the substrate is first decontaminated to deactivate microorganisms and enzymes, which could attack the biomolecules which are to be immobilized. Next. a silsesquioxane coating is formed on the substrate, and then biomolecules are immobilized on the coated substrate. In a typical application of the invention, after coating of the substrate with a silsesquioxane and prior to immobilization of the biomolecule, substrates are packaged for storage and shipping. The packaging should be free of dust and should not generate dust during loading of the substrate in the packaging of. or during storage of the substrate in the packaging, or as a result of handling of the packaging during shipment of the substrate to the end user.

As discussed above, variabiliy in spot size and high background levels can be problematic in biomolecule hybridization. Variability in spot size and high background levels can arise from non-uniformities in a slide's coating which, in turn. can result in a working surface whose hydrophilic/hydrophobic properties are non-uniform.

Since biomolecules are typically applied to a slide in an aqueous solution, the hydrophilic/hydrophobic properties of the working surface may determine the quantities of the biomolecules which are deposited. In addition, the properties of the surface determine whether or not the deposited biomolecules stay at exactly those locations where they are deposited or possibly move slightly away from those locations. for example, possibly move towards a part of the surface which is. for example, slightly more hydrophilic. The present invention provides a uniform surface, and its uniform wettability ensures that printed DNA solution spots remains where they are printed and a have a round shape. In addition, the present invention provides a uniform coating.

Various techniques are known in the art for immobilizing DNA and oligonucleotides on surfaces, essentially any of which can be used in the practice of the invention. A discussion of representative immobilization techniques used in the art can be found in U.S. Patent Number 5.919.626 and the references listed in that patent. Similarly. immobilization techniques are known for other biomolecules. such as specific binding members. Along the same lines, techniques for immobilization of molecules useful in tissue culture systems, e.g.. collagen, are also well-known in the art. It is understood that surfaces produced in accordance with the present invention can be used to immobilize a variety of biomolecules including, but not limited to DNA arrays. oligonucleotides. protein arrays and cell arrays.

The process steps of the invention described above can be carried out using conventional equipment known in the art. e.g.. equipment commonly found in a chemistry laboratory. Without intending to limit the invention in any manner, the present invention will be more fully described by the following examples.

### EXAMPLE

### Cleaning and Coating of Slides

Glass slides were first cleaned as follows. The glass slides are preferably ESCO™ (Erie Scientific Co.. Portsmouth. NH) soda lime glass slides. Throughout the experiment. the slides were handled with plastic or Teflon™ tweezers and processed and stored in plastic or Teflon™ beakers. The slides were cleaned in a 50/50 (by volume) mixture of hydrogen peroxide (30% electronic grade) and sulfuric acid (95%-98% electronic grade). The slides were dipped in the solution for about 20 minutes and then rinsed using ultra pure milli-Q water. The slides were then stored in a bath of ultra pure milli-Q water to avoid contamination.

Prior to applying a coating to the slides, they were removed from the bath of water. A solution of silsesquioxane solution was prepared using water-borne silsesquioxane oligomer solutions available from Gelest. Inc. Tullytown. Pennsylvania. Gelest product codes WSA-7011. WSA-9911. and WSA-7021 were used. each of which contained approximately 25 weight percent silsesquioxane in solution. The silsesquioxane solutions were diluted with water to about 2 weight percent. Cleaned glass slides were dipped in the silsesquioxane solutions for about 15 to 40 minutes. Each of the slides was rinsed by dipping them in three separate beakers of ultra pure milli-Q water for at least one minute. After rinsing, the slides were dried with nitrogen flowing through an anti-dust filter.

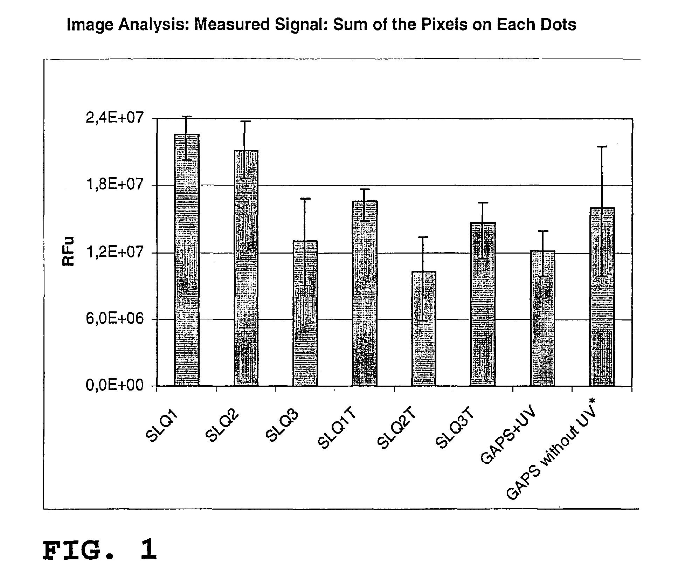

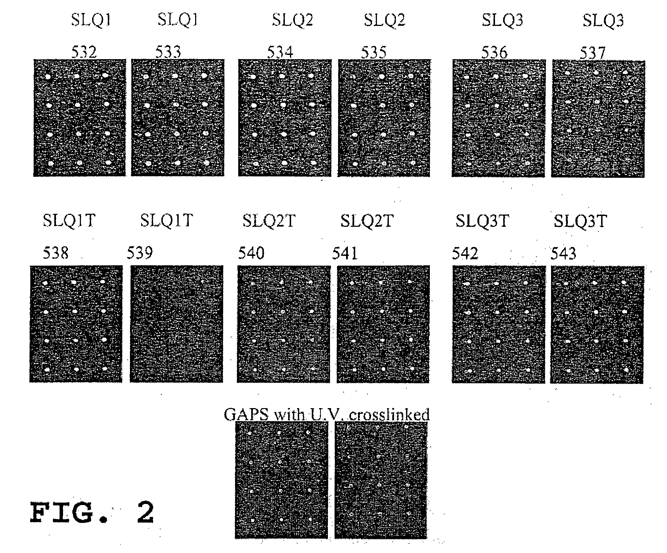

Several slides were prepared as follows. Three different groups of slides were dipped in three different silsesquioxane solutions. Accordingly, one group of slides was coated with a first silsesquioxane solution having the general formula: 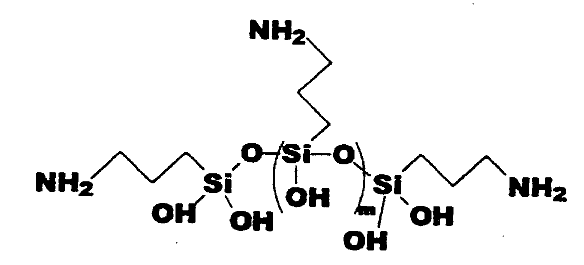 One subgroup of these slides was stored for later experimentation. The results for these slides are labeled SLQ1 in Figures 1 and 2. Another subgroup of these slides was heated for one hour at about 110° C for about one hour and stored for later experimentation. The results for these slides are labeled SLQ1T in Figures 1 and 2.

A second group of slides was dipped in a silsesquioxane solution having the general formula: 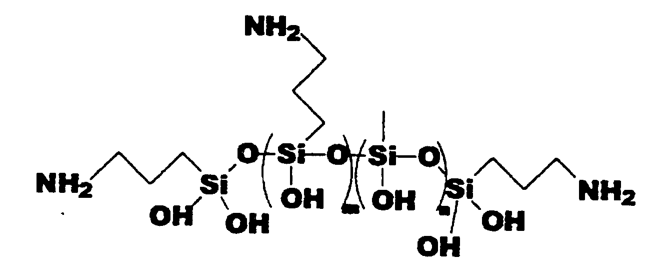 One subgroup of these slides was stored for later experimentation. The results for these slides are labeled SLQ2 in Figures 1 and 2. Another subgroup of these slides were heated for one hour at about 110° C for about one hour and stored for later experimentation. The results for these slides are labeled SLQ2T in Figures 1 and 2.

A third group of slides was dipped in a silsesquioxane solution having the general formula:

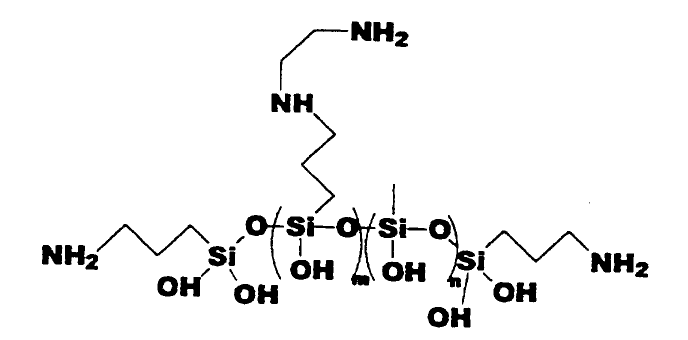

One subgroup of these slides was stored for later experimentation. The results for these slides are labeled SLQ3 in Figures 1 and 2. A second subgroup of these slides was heated for one hour at about 110° C for about one hour and stored for later experimentation. The results for these slides are labeled SLQ3T in Figures 1 and 2.

A fourth group of slides was CVD coated with GAPS according to the following procedure. A clean, room temperature slide was placed in the presence of a hot silane vapor produced from pure silane liquid heated to 60° C. Silane vapor condensed onto the surface of the slide. One subgroup of these slides with the silane coating was then treated under a UV lamp at a wavelength of about 260 nm and an energy of 60 mJ/cm². This sample is labeled as "GAPS + UV" in Figures 1 and 2. The GAPS coated sample that was not treated with UV light is labeled "GAPS without UV" in Figure 1.

All slides were then stored in plastic boxes under normal atmospheric conditions until use in printing and hybridization experiments.

### Printing and Hybridization

This example illustrates the use of slides prepared in accordance with the invention to immobilize biomolecules. The uniformity of DNA retention and hybridization may be demonstrated with the printing of a DNA solution, followed by the hybridization of the immobilized DNA with a target probe. The printed DNA is unmarked and the target DNA (from the same gene as the printed DNA) is marked with, for example. Amersham's Cy3 fluorescence marker. An array of 3 X 4 spots was printed on each slide.

Prior to printing, the DNA probe was prepared with three steps. First, the probe (cDNA or PCR-based) was brought up to 15 µL in 3X Saline Sodium Citrate (SSC). 0.1% Sodium Dodcyl Sulfate (SDS) with 10 µg polyadenine (10 mg/mL stock: Pharmacia. ref. 27-4110-01) and 10 µg *E. coli* tRNA (4 mg/mL stock. Boehringer Mannheim. ref 109 550). Second, the probe was denatured by boiling it in a heat block for between 3 to 5 minutes. Third, the probe was snap-cooled in ice.

Printing the DNA probe was performed on a noncommercial robot with movement capabilities in the X. Y and Z directions. A piezo head (MDK-140H. Microdrop GmbH.. Norderstedt. Germany) was used for the printing. The noncommercial robot actuated the piezo for 0.03 seconds for a nanoliter volume range of dispensation. The slide was then subjected to chemical blocking, denaturation, pre-hybridization. and hybridization following conventional procedures. The gene CYS3 was used for the DNA in both the printing and the hybridization solutions.

The materials used for hybridization were succinic anhydride (Aldrich Inc. ref. 13.4441-4). n-methyl-2-pyrrolidinone (Aldrich Inc. ref. 32.863-4). boric acid (Gibco BRL. ref. 15583-024). 20X SSC (Sigma Inc. ref. S6639). SDS (Amresco. ref. 0837). and absolute ethanol (Merck. ref. 1 000983).

Next. the slides were prepared for hybridization with three steps. First, the slides were snap dried on a 100°C hot plate for three seconds. Second, the slides were soaked in a blocking solution for 15 minutes made from (70 millimole) succinic anhydride in a solution consisting of 315 ml of n-methyl-2-pyrrolidinone and 35 ml of 0.2 M boric acid (pH 8). Third, the slides were denatured in water at 95°C for 2 minutes, followed by an ethanol rinse, and dried with nitrogen gas.

The slides were then hybridized in three steps. First, the target was pipetted onto the slides and a drop cover slip was placed onto the liquid. Second, the slides were assembled in the hybridization chamber and clamped shut. Third, the slides were submerged in a 50°C water bath for approximately 12 hours.

Finally, the slides were cleaned by removing the coverslip with tweezers. placing the slides in a 0.1X SSC. 0.1% SDS at room temperature for between 5 to 10 minutes, transferring the slides to 0.1X SSC for between 5 to 10 minutes and drying the slides with nitrogen gas.

The hybridized slides were then scanned on the Scan Array 3000 (General Scanning Inc.. Watertown, MA) set to laser 100 and PMT 95. The spots were then quantified with Optimas™ (Optimas Corp.. Bothell. WA) software by delineating the spot according to a threshold value and calculating the mean gray signal within the defined surface. The results from the Scan Array 3000 are shown in Figure 1.

The hybridization results were then obtained by reading the fluorescence after excitation with a laser at about 543 nm or at about 633 nm depending on the type of fluorophone in the DNA strand. The results are shown in Figure 2.

### Results

As Figure 1 shows the slides coated with silsesquioxanes SLQ1 and SLQ2 have an average Rfu signal that is approximately 1.5 times greater than the GAPS coated slide without UV treatment. The slides coated with SLQ3 have an average Rfu signal that is slightly greater than the GAPS-coated slide without UV treatment.

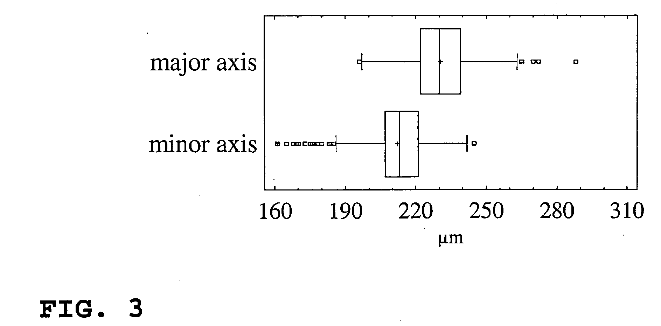

Experimentation has also indicated that the slides coated with silsesquioxanes in accordance with the present invention provides a reproducible spot size, The spots on the silsesquioxane coated slides are circular and uniform in diameter as shown by Figure 2. Statistical analysis of the spots on the silsesquioxane coated slides was performed using STATGRAPHICS® software. The results of this analysis is shown in Fig. 3. Fig. 3 shows that the average values (indicated by the cross inside the boxes) and the median values (indicated by vertical crossbars outside the boxes) of the major axis and the minor axis of the dots are approximately equal. The size distribution (indicated by the boxes) of the major and minor axis show that silsesquioxanes produce reproducible patterns on glass slides.

In addition, the coatings exhibited excellent durability when stored under normal conditions and measured for Rfu after storage. Slides prepared in accordance with the Example were stored for two and six month period and a hydridization experiment was performed on new slides and the stored slides in accordance with the Example. The Rfu signal from the slides stored for two and six months were within 10-15% of the Rfu signal measured from freshly prepared slides.

It will be apparent to those skilled in the art that various modifications and variations can be made to the present invention without departing from the spirit or scope of the invention. For example, a variety of silsesquioxanes and combinations of silsesquioxanes containing various functional groups appropriate for biomolecule immobilization may be used in accordance with the present invention. Thus. it is intended that the present invention cover modifications and variations of this invention provided they come within the scope of the appended claims and their equivalents.

## Claims

1. A method of immobilizing biomolecules on a surface of a substrate comprising coating the substrate with a silsesquioxane solution and immobilizing the biomolecules on the silsesquioxane coated surface.

2. The method of claim 1. wherein the silsesquioxane is a silsesquioxane oligomer.

3. The method of claim 2. wherein the silsesqioxane oligomer is in an aqueous solution.

4. The method of claim 3. wherein the silsesquioxane oligomer has an average molecular weight of from about 150 to about 1000.

5. The method of claim 3. wherein the silsesquioxane oligomer has an average molecular weight of from about 200 to about 700.

6. The method of claim 3, wherein the silsesquioxane oligomer has an average molecular weight ranging of from about 250 to about 650.

7. The method of claim 2. wherein the oligomer is an aminoalkylsilsesquioxane, wherein the alkyl group is a C₂ or higher alkyl group.

8. The method of claim 2. wherein the oligomer is an aminopropylsilsesquioxane oligomer.

9. The method of claim 2. wherein the oligomer is an aminopropylsilsesquioxane-methylsilsesquioxane copolymer oligomer.

10. The method of claim 7. wherein the biomolecules are nucleic acids or oligonucleotides.

11. The method of claim 10. wherein the silsesquioxane coating is applied in an aqueous solution.

12. A substrate having biomolecules immobilized thereon produced by the method of claim 1.

13. A substrate having biomolecules immobilized thereon produced by the method of claim 7.

14. A substrate having biomolecules immobilized thereon produced by the method of claim 8.

15. A substrate having biomolecules immobilized thereon produced by the method of claim 9.

16. A substrate having biomolecules immobilized thereon produced by the method of claim 10.

17. The substrate of claim 12. wherein the biomolecules are nucleic acids or oligonucleotides.

18. The substrate of claim 17. wherein the substrate is a high density array.

19. The substrate of claim 18, wherein the substrate material is selected from the group consisting of glass, quartz and silica.

20. A method of coating a substrate for the immobilization of a biomolecule comprising coating the substrate with a solution including an aminoalkylsilsilsesquioxane oligomer including a C₂ or higher alkyl group, the oligomer having a molecular weight of from about 200 to 700.

21. The method of claim 20, wherein the coating step includes dipping the substrate in an aqueous solution.

22. The method of claim 20, wherein the oligomer is selected from the group consisting of aminopropylsilsesquioxane oligomer. aminopropylsilsesquioxane-methylsilsesquioxane copolymer. and mixtures thereof.

23. A method of coating a substrate for the immobilization of a biomolecule comprising coating the substrate with a solution including a selected from the group consisting of a silsesquioxane described by the formulae: 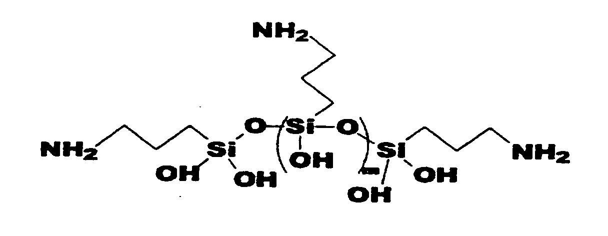 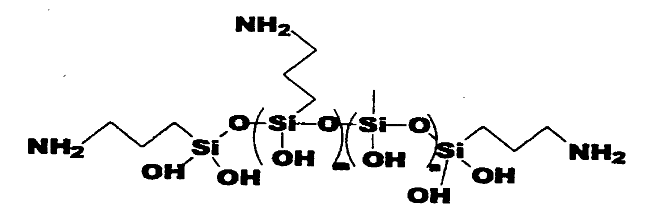 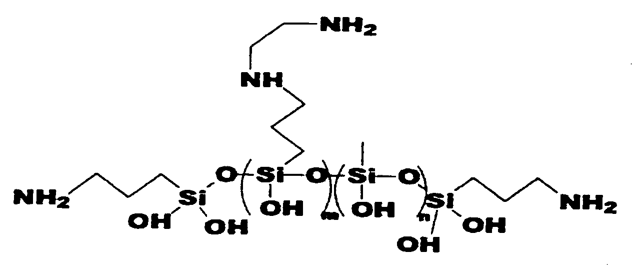 and mixtures thereof, wherein m and n are integers greater than or equal to 1 such that the average molecular weight of the resulting silsesquioxane oligomer ranges from about 150 to about 1000.
